(19) Europäisches Patentamt
European Patent Office
Office européen des brevets

(11) Publication number: **0 124 659**
**A1**

(12) # EUROPEAN PATENT APPLICATION

(21) Application number: 83302077.9

(22) Date of filing: 13.04.83

(51) Int. Cl.³: **A 61 F 1/00**
A 61 L 17/00

(43) Date of publication of application:
**14.11.84 Bulletin 84/46**

(84) Designated Contracting States:
**DE FR GB IT NL SE**

(71) Applicant: **KOKEN CO. LTD.**
**5-18 Shimoochiai 3-chome**
**Shinjuku-ku Tokyo(JP)**

(72) Inventor: **Miyata, Teruo**
**6-29, Shimoochiai 3-chome**
**Sinjuku-ku Tokyo(JP)**

(72) Inventor: **Noishiki, Tasuharu**
**Yamada Misasa-cho**
**Touhaku-gun Tottori-ken(JP)**

(74) Representative: **Crampton, Keith John Allen et al,**
**D YOUNG & CO 10 Staple Inn**
**London WC1V 7RD(GB)**

(54) **Medical material.**

(57) Medical material contains collagen that has been chemically modified by succinylation of terminal $-NH_2$ groups of the side chains attached to poly peptide chains of the collagen, so that the $-NH_2$ groups are converted into groups having -COOH groups. This succinylation can be carried out by reacting succinic anhydride with the $-NH_2$ groups of the collagen. Since the above medical material has excellent compatibility with living bodies, especially blood, it is suitable for use as a replacement material for tissues and/or organs which are kept in contact with blood at their surfaces, e.g. in artificial blood vessels, artificial valves and some parts of artificial hearts that are kept in contact with blood, and as a patching material for hearts.

EP 0 124 659 A1

Croydon Printing Company Ltd.

# MEDICAL MATERIAL

This invention relates to medical material compatible with living bodies and especially with blood.

Collagen is the principal component of connecting tissues of mammals, such as skins, blood vessels, bones, fasciae and teeth. It serves to protect animals from irritation from the outside and to maintain body configurations and, at the same time, it also serves as a supporting stromata for a variety of cells. Collagen accounts for one third of the whole proteins of mammals and is thus available in abundance. It has therefore found commercial utility in various industrial fields such as the leather, sausage-casing and gelatin industries. In recent years, it has also come into the limelight as a medical material. Being different from artificial medical materials such as synthetic polymers and ceramics, collagen inherently makes up living bodies and, as a corollary to this, has various characteristic properties:-

(a) Owing to its proteinaceous nature, it is of course antigenic. However, its antigenicity is very weak compared with that of other proteins and its principal antigenic determinant is the telopeptido residue, which may be removed by treating collagen with a protease such as pepsin. Thus, its antigenicity can be reduced by such pepsin treatment to such an extent that no substantial propblem would occur. Its antigenicity may be reduced further by subjecting it to tanning (crosslinking) treatment, e.g. using glutaraldehyde.

(b) When implanted in a body, collagen gradually replaces a part of the connecting tissue and, as a result, is absorbed in the connecting tissue. Thereafter, it is excreted as amino acids and peptides in urine. Since this absorption speed may be lowered by the tanning (crosslinking) treatment, it is possible to control the absorption speed by controlling the tanning treatment.

(c) Owing to its excellent performance as a footing for the growth of cells, application of collagen has the effect of expediting the curing of a wound. Collagen enjoys excellent compatibility with tissues in living bodies.

(d) When collagen is brought into contact with blood, it induces platelet agglutination and subsequently development of thrombus. Therefore, it may be used as a medical material for the purpose of obtaining a haemostatic effect.

(e) Since collagen purified from bones induces and promotes osteogenesis, it is also used as an osteogenetic medical material for bone deficiency diseases.

As collagen has the properties of cooperating with living bodies as described above, it is used as a substitute for many tissues such as the base of artificial skins, artificial blood vessels, artificial bones, haemostatics, artificial valves, patching materials, artificial tympanic membranes, and hybrid artificial organs, and as material for contact

lenses and drug carriers.

When using collagen as a blood vessel material, it is most common to chemically treat an animal blood vessel and then use the thus-treated blood vessel as an artificial blood vessel. However, collagen promotes the platelet agglutination reaction and forms thrombus when brought into contact with blood, leading to frequent occurrence of clotting of artificial blood vessels in the initial periods of implantation. However, when the formation of thrombus has occurred rather little in the initial period, the thrombus layer will be gradually absorbed and, instead, endothelia will come out from the interior of the living body and will eventually cover up the surface of the artificial blood vessel (i.e., conversion into a pseudo- and endomembrane). Once the artificial blood vessel has been covered by such a pseudo- and endomembrane, thrombus will no longer be formed and the artificial blood vessel will serve just like ordinary blood vessels. Accordingly, it is most important for a collagen-containing artificial blood vessel to minimize the development of thrombus in the initial period of implantation and to promote the conversion into a pseudo- and endomembrane.

One of the present inventors, Dr. Teruo Miyata, reported in "Deposition of Platelets and Fibrin on Chemically Modified Collagen Hollow Fiber", Trans. Amer. Soc. Artif. Int. Organs, 22, 261(1976) that the surface of succinylated collagen has anticoagulant property. Namely, hemeral venous blood was passed through a collagen capillary and the surface of the

collagen capillary was observed as to adhesion and agglutination of platelets, deposition of fibrin, etc. The above observation indicated that succinylated collagen produced extremely little deposition of fibrin and far more antithrombotic compared with unsuccinylated collagen. However, it was necessary to determine if the effects of the succinylation would also be brought about in vivo as the above investigation was carried out neither in vivo nor in vitro but in-between, namely, ex-vivo.

On the other hand, another one of the present inventors, Dr. Yasuharu Noishiki, proposed a connective tissue tube making use of a "TETORON" net in "Alcohol-Preserved Homo- and Heterologus Connective Tissue Tube for Arterial Prosthesis", Artif. Organs, 2 (suppl.), 152(1977). The above connective tissue tube was formed by inserting the tube subcutaneously in an animal so that a connecting tissue consisting principally of collagen would cover all over the tube. The connective tissue tube was implanted in the thoracic descending aorta of a dog. Even shortly after the implantation (i.e., a few hours later), a thick thrombus layer having a thickness of 20 - 100 μm and including solid blood components entrapped therein was formed on the connective tissue tube. This thrombus was absorbed little by little and endothelia came out. This appearance of endothelia, however, took place about 4 weeks after the implantation. Thus, the curing was very slow.

In accordance with this invention, a medical material comprises collagen which has been chemically modified by succinylation of terminal $-NH_2$ groups of side chains attached to polypeptide chains of the collagen so that the $-NH_2$ groups are converted into groups having $-COOH$ groups. Such materials, as will appear from the description below of preferred embodiments, can be excellent in compatibility with living bodies, especially with blood in living bodies, thereby exhibiting a good capacity for forming pseudo- and endomembranes when applied as artificial blood vessels and thus being extremely effective in accelerating curing speeds.

One example of the medical material according to this invention is formed of a base, which is in turn made of such a synthetic polymer as will be described below, and succinylated collagen which covers the base and will also be described below. The synthetic polymer may be a polyester such as "Tetoron" or "Goatex". While a base made of such a synthetic polymer is kept embedded subcutaneously in an animal, the connecting tissue - which consists principally of collagen - of the animal is caused to build up to a thickness of 100 to 1,000 μm on the base. The above-mentioned connective tissue tube can be obtained by forming a layer of a connecting tissue on a tube made of the "Tetoron" net as described above. The layer of the connecting tissue is formed as if it fills up the meshes of the tube and covers it up entirely. The tube of "Tetoron" net, which is present in the connective tissue tube, has a thin and porous wall and thus permits capillaries to penetrate readily through it from the surrounding tissue. This is certainly desirable in order to promote the conversion of the tube into a pseudo- and endomembrane. Accordingly, the above tube is particularly suitable as a starting material for making medical material according to this invention.

Certain tissues or organs forming living bodies such as cow carotid arteries, human umbilical veins and pig ureters can also be suitably used as starting materials for medical materials according to this invention. In the case of these tissues and organs forming living bodies, it is generally necessary to succinylate the collagen contained in such organs and tissues after treating them with a protease such as trypsin or papain to remove unnecessary cell components.

The succinylation of collagen may be conducted by treating the above-mentioned artificial organs or a tissue or organ forming a living body with an acetone or alcohol solution of succinic anhydride. In the course of the treatment, certain $-NH_2$ groups of the polypeptide chain of collagen undergo a reaction with succinic anhydride and are converted into $-NHCOCH_2CH_2COOH$ groups, that is, are chemically modified into groups containing -COOH groups, as shown by the following formula:

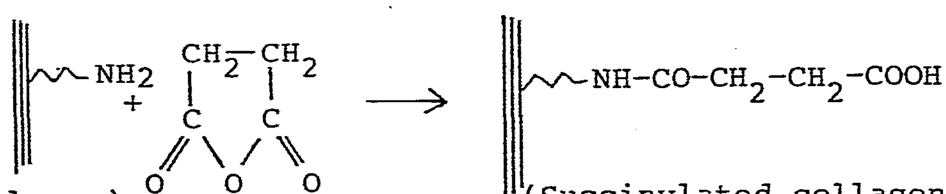
(Collagen)

Succinic anhydride

(Succinylated collagen)

The isoionic point of collagen is lowered from about 8 to about 4.5 by the above succinylation. The succinylation causes collagen to contain more water, thereby lowering the strength of the collagen. To maintain its strength, it may be possible to control the succinylation reaction so as to subject only the surface area of a collagen-containing medical material to succinylation so that the $-NH_2$ groups present inside the material are able to take part in a tanning (crosslinking) reaction. By doing so, it is feasible to successfully carry out a tanning (crosslinking) treatment relying upon $-NH_2$ groups in order to make the collagen tissue physically stronger and resistant to its absorption after its implantation. The tanning (crosslinking) treatment may be carried out, for example, by reacting an aldehyde, such as glutaraldehyde, or its analogue, with $-NH_2$ groups. Here, the percentage of succinylated $-NH_2$ groups to the remaining unsuccinylated $-NH_2$ groups may be calculated with respect to the entire collagen. Expressing the above-calculated value in terms of the degree of succinylation of collagen, the degree of succinylation is preferably 50% or higher. If it is lower than 50%, the effects of the succinylation will not result to any satisfactory extent.

In artificial blood vessels made of synthetic material, for example, of "Tetoron" or of "Goatex", it is very effective to apply a solution of succinylated collagen on the inner walls of the artificial blood vessels to enhance their compatibility with blood and, in view of the usual network structures of these artificial blood vessels, to avoid leakage of blood through their meshes. Medical materials that have been coated with succinylated collagen at places where they are kept in contact with blood are considered to have commercial value. The coating may be effected, for instance, by succinylating collagen that has previously been rendered soluble with pepsin and then coating the thus-treated collagen to a thickness of 10 - 20 µm. Needless to say, this coating technique may similarly be applied to medical materials that have been obtained using natural tissues such as cow carotid arteries, human umbilical veins and pig ureters. Connecting tissues of animals useful for the production of medical materials should not be limited to tubular tissues such as blood vessels or ureters. The paricardium is a sheet-like membrane and is thus useful as artificial valve material, a lining material for artificial hearts (applied at places where the hearts are kept in contact with blood) and a patching material. The paricardium per se is valuable

material as it may be subjected to succinylation.

The invention will be described in further detail in the following Examples, which are illustrative.

Example 1:

A tube having an inner diameter of 7 mm and a length of 5.7 cm was prepared with a "TETORON" net. It was kept inserted subcutaneously for 20 days in a matured dog and then taken out, with connecting tissues of the dog attached around the tube. It was thereafter treated overnight with a phosphoric acid buffer solution (pH 7.0) containing 0.01% of ficin. After washing the thus-treated tube with physiological saline, it was immersed in 500 ml of a 0.02-M borax buffer solution (pH 9.0), followed by gradual addition of 100 ml of a 5% acetone solution of succinic anhydride while maintaining its pH always at 9 with NaOH. Subsequent to the incorporation of succinic anhydride, the resultant mixture was allowed to stand overnight at pH 9.0. Then, the thus-conditioned connective tissue tube was washed with water and then dipped in a 70% alcohol to sterilize it. After removing the alcohol with a germ-free physiological saline, the connective tissue tube was implanted in the thoracic descending aorta of a dog. It was observed for more than one month from immediately after the implantation. Unsuccinylated connective tissue tubes were also used as a control group.

The above observation was effected by eye, optical microscope and scanning electron microscope. In the case of succinylated connective tissue tubes, no red thrombus was

observed and thin layers each having a thickness in the range of 5 - 20 μm and consisting solely of fibrin were formed. In addition, development of endothelia was observed one week after implantation. The growth of endothelia was fast and endothelia were allowed to spread substantially all over the surfaces of the tubes upon an elapsed time of one month, thereby completing the conversion of the tubes into pseudo- and endomembranes. Contrary to the above-mentioned succinylated connective tissue tubes, those of the control group were accompanied by the formation of thick layers of red thrombus and their conversion into pseudo- and endomembranes were delayed by 3 - 4 weeks. As illustrated above, succinylated connective tissue tubes are capable of avoiding thrombus formation in the initial period and permit the curing to proceed at faster speeds.

Example 2:

Surrounding adipose tissue was removed from a pig ureter and the thus-prepared pig ureter was immersed overnight in a phosphoric acid buffer solution (pH 7.0) containing 0.01% ficin. After thoroughly washing the resultant pig ureter with water, it was dipped in 500 ml of a 0.02-M borax buffer solution (pH 9.0), followed by a gradual addition of 100 ml of a 5% acetone solution of succinic anhydride while maintaning the resulting mixture always at pH 9.0 with NaOH. After holding the thus-conditioned pig ureter for 24 hours at pH 9.0, it was thoroughly washed and then sterilized with a 70% alcohol. The thus-succinylated ureter was implanted in the thoracic

descending aorta of a dog. Its comparison with a control group (which had not been subjected to the succinylation treatment) showed that, in the case of the succinylated ureter, no red thrombus was formed and development of endothelia took place faster than that in the control group whereas red thrombus was formed in the initial period and conversion into pseudo- and endomembranes was delayed in the control group. It was discovered that a succinylated ureter is an excellent material and can thus be used as an artificial blood vessel, similar to the succinylated connective tissue tube of Example 1.

Example 3:

The paricardium of a cow was washed with physiological saline and then treated overnight with a phosphoric acid buffer solution (pH 7.0) containing 0.01% of ficin. Subsequent to its washing with water, the thus-prepared paricardium was immersed in 500 ml of a borax buffer solution (0.02 M ; pH 9.0), followed by an addition of 100 ml of a 5% acetone solution of succinic anhydride. The resultant mixture was adjusted to pH 9.0, where the succinylation treatment was allowed to take place for 10 minutes. After washing the thus-treated paricardium with water, it was dipped in a phosphoric acid buffer solution (pH 7.4) containing 0.62% of glutaraldehyde to conduct its tanning and sterilization at the same time. The succinylation degree of the thus-prepared paricardium was 55%. Namely, 55% of all the anino groups present in the paricardium have been succinylated. Thus, the paricardium had been succinylated principally at its surfaces and its interior

$-NH_2$ groups had been subjected to the tanning treatment with glutaraldehyde. Such a paricardium may be utilized for preparing artificial valves, lining artificial hearts or patching hearts. It is a material having excellent compatibility with blood and, at the same time, exhibiting sufficient strength even after its implantation in a body and undergoing no ready absorption or digestion.

Example 4:

The cow dermis was comminuted and, subsequent to its washing with a 5% salt water, washed with water, added with water to adjust the collagen concentration to about 3%, and finally adjusted to pH 3 with HCl. Pepsin was added to the thus-prepared solution in the amount of 1% based on collagen present therein. The resultant mixture was maintained at 10 - 25°C for 3 - 6 days, thereby causing collagen fibers to dissolve and thus obtaining a viscous collagen solution (the collagen solubilized with pepsin will hereinafter be called "atherocollagen"). By adjusting the solution to pH 7 - 8 with NaOH, a precipitate of atherocollagen occurred. The precipitate was collected by centrifugation and, subsequent to its washing with water, water was added thereto to give a collagen concentration of 1% and the pH of the resultant aqueous mixture was adjusted to pH 9.0. Then, a 5% acetone solution of succinic anhydride was added little by little with stirring to the above aqueous mixture while maintaining its pH always at 9.0 with NaOH. Use of succinic anhydride at a ratio of 0.5 relative to the dry weight of collagen will provide a

succinylation degree of collagen of 85% or higher. After allowing the mixture to stand overnight at pH 9.0, it was adjusted to pH 4.5 to form a precipitate of succinylated atherocollagen. It was collected by centrifugation, washed with water and then purified. Since the isoionic point (pI) of the succinylated atherocollagen was 4.5, it precipitates at pH 4.5 but is dissolved in the acidic pH ranges lower than pH 4 and higher than pH 5.0.

Then, a 1% aqueous solution (pH 6.5 - 7.5) of succinylated atherocollagen was prepared and coated on an artificial blood vessel made of "TETORON". After its coating, the artificial blood vessel was dried in wind and exposed for one hour to ultraviolet rays from a 20-W germicidal ultraviolet lamp placed 10 cm apart from the blood vessel so as to induce crosslinking in the thus-coated succinylated atherocollagen. Artificial blood vessels made of a synthetic polymer and coated with succinylated collagen as described above will be free of blood leakage, show improved compatibility with blood and promote the formation of pseudo- and endomembranes thereon, thereby ensuring faster curing.

## CLAIMS

1. A medical material comprising collagen, characterized in that the collagen has been chemically modified by succinylation of terminal $-NH_2$ groups of side chains attached to polypeptide chains of the collagen so that the $-NH_2$ groups are converted into groups having -COOH groups.

2. Medical material according to Claim 1 that consists principally of collagen that has been succinylated only on the surface of the material and not subjected to succinylation in the interior of the material.

3. Material according to Claim 2, in which the degree of succinylation is 50% or higher.

4. Material according to Claim 2 or 3, in which the $-NH_2$ groups of collagen present in the interior of the material have been crosslinked by an aldehyde or an analogue of an aldehyde.

5. Medical material according to Claim 1 formed of a base on which a coating layer of succinylated collagen is formed.

6. Material according to Claim 5, in which the thickness of the coating layer is from 10 µm to 20 µm.

7. Material according to Claim 5 or 6, in which the base is made of a natural tissue or organ.

8. Medical material according to Claim 1 formed of a base of synthetic polymer and a connecting tissue covering the base, the collagen present in the connecting tissue having been succinylated.

9. Material according to Claim 8, in which the base is formed of a tube of a "Tetoron" net.

10. Material according to Claim 8 or 9, in which the thickness of the connecting tissue is 100 to 1,000 μm.

11. Material according to Claim 8, 9 or 10, in which the connecting tissue has been formed by subcutaneously implanting the base made of synthetic polymer in an animal.

12. Medical material according to Claim 1 made of a natural tissue or organ in which the collagen present has been succinylated at least on the surface of the tissue.

13. Material according to Claim 12, in which the natural tissue is a blood vessel, a ureter or a paricardium.

14. Material according to any one of Claims 1 to 13, in which the isoionic point of the succinylated collagen is about 4.5.

15. Material according to any one of Claims 1 to 14, in which the succinylated collagen has been obtained by reacting succinic anhydride with collagen.

European Patent Office

# EUROPEAN SEARCH REPORT

0124659

Application number

EP 83 30 2077

## DOCUMENTS CONSIDERED TO BE RELEVANT

| Category | Citation of document with indication, where appropriate, of relevant passages | Relevant to claim | CLASSIFICATION OF THE APPLICATION (Int Cl 3) |
|---|---|---|---|
| X | US-A-3 927 422 (P.N. SAWYER)<br>* Claims 1,6-9; column 6, lines 45-64 * | 1-8,10-15 | A 61 F 1/00<br>A 61 L 17/00 |
| Y | | 9 | |
| | --- | | |
| X | US-A-4 082 507 (P.N. SAWYER)<br>* Column 3, lines 54-68; claim 1 * | | |
| | --- | | |
| X | EP-A-0 037 381 (SOLCO)<br>* Page 7, lines 1-3 * | 1,5,6 | |
| | --- | | |
| A | EP-A-0 000 949 (P.N. SAWYER)<br>* Claims 3,6,12 * | 1 | TECHNICAL FIELDS SEARCHED (Int. Cl. 3) |
| | --- | | |
| Y | GB-A-1 183 497 (ETHICON)<br>* Claim 2; example III * | 9 | A 61 F 1/00<br>A 61 F 1/24<br>A 61 L 17/00 |
| | --- | | |
| A | FR-A-2 265 345 (W.L. GORE)<br>* Page 5, lines 11-19 * | 9 | |
| | ----- | | |

The present search report has been drawn up for all claims

| Place of search | Date of completion of the search | Examiner |
|---|---|---|
| THE HAGUE | 16-12-1983 | PELTRE CHR. |

CATEGORY OF CITED DOCUMENTS

X : particularly relevant if taken alone
Y : particularly relevant if combined with another document of the same category
A : technological background
O : non-written disclosure
P : intermediate document
T : theory or principle underlying the invention
E : earlier patent document, but published on, or after the filing date
D : document cited in the application
L : document cited for other reasons
& : member of the same patent family, corresponding document